# EUROPEAN PATENT SPECIFICATION

(11) **EP 3 709 891 B1**
(45) Date of publication and mention of the grant of the patent: **22.03.2023**
(21) Application number: 18799732.5
(22) Date of filing: 06.11.2018
(51) Int. Cl.: A61B 8/12, A61B 8/08, A61B 8/00, G01S 15/89

(54) **ICE CATHETER WITH MULTIPLE TRANSDUCER ARRAYS**
KATHETER ZUR INTRACARDIALEN ECHOKARDIOGRAPHIE MIT MEHREREN WANDLERARRAYS
CATHÉTER À ÉCOGRAPHIE INTRACARDIAQUE COMPORTANT DE MULTIPLES RÉSEAUX DE TRANSDUCTEURS

(30) Priority: 14.11.2017 EP 17201574
(43) Date of publication of application: 23.09.2020
(73) Proprietor: Koninklijke Philips N.V., 5656 AG Eindhoven (NL)
(72) Inventor: KOLEN, Alexander, Franciscus, 5656 AE Eindhoven (NL); JANSE, Cornelis Pieter, 5656 AE Eindhoven (NL); BELT, Harm Jan Willem, 5656 AE Eindhoven (NL); SCHMEITZ, Harold Agnes Wilhelmus, 5656 AE Eindhoven (NL); SMEETS, Bart Leonardus Martinus, 5656 AE Eindhoven (NL); VAN HEESCH, Franciscus Hendrikus, 5656 AE Eindhoven (NL)
(74) Representative: Philips Intellectual Property & Standards
(86) International application number: PCT/EP2018/080301
(87) International publication number: WO 2019/096628

(56) References cited:
- US-A- 5 876 345
- US-A1- 2004 044 284
- US-A1- 2005 215 895
- US-A1- 2007 167 823
- US-A1- 2013 131 499
- US-A1- 2015 080 727
- CHENG YAO ET AL: "Spatial compounding of large numbers of multi-view 3D echocardiography images using feature consistency", BIOMEDICAL IMAGING: FROM NANO TO MACRO, 2010 IEEE INTERNATIONAL SYMPOSIUM ON, IEEE, PISCATAWAY, NJ, USA, 14 April 2010 (2010-04-14), pages 968-971, XP031693430, ISBN: 978-1-4244-4125-9
- CHENG YAO: "Spatial compounding of large sets of 3D echocardiography images", SPIE, PO BOX 10 BELLINGHAM WA 98227-0010 USA, 2009, XP040495336,
- GANG GAO ET AL: "Real-time compounding of three-dimensional transesophageal echocardiographic volumes: The phantom study", PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMEDICINE, EMBC 2009, IEEE, 3 September 2009 (2009-09-03), pages 499-502, XP031881461, DOI: 10.1109/IEMBS.2009.5333122 ISBN: 978-1-4244-3296-7
- KRISTOF RALOVICH: "Spatial Compounding of 2D and 3D Volume ICE, based on CatheterPose Tracking from Fluoroscopy", PRIOR ART PUBLISHING, 2014, pages 214-216, XP040638683,

## Description

### FIELD OF THE INVENTION

The invention relates to an ultrasound image compounding method for use with an Intra Cardiac Echography, i.e. ICE, catheter. The invention finds application in the field of cardiac medical procedures.

### BACKGROUND OF THE INVENTION

ICE is widely used during interventional cardiac procedures to visualize anatomical features such as the atrial septum, the aortic valve, pulmonary veins and so forth. ICE is also used to image interventional devices such as ablation catheters and lasso catheters that are used in performing medical procedures on the heart. An ICE catheter includes an array of ultrasound transducer elements which is used to generate an ultrasound image. Conventionally, ICE catheters use a one-dimensional array of ultrasound elements to generate a two-dimensional image slice. Such two-dimensional image slices have a somewhat limited field of view, and so ICE catheters that use a two-dimensional array of ultrasound elements to generate a three-dimensional, i.e. volumetric, image have also been developed.

Ultrasound images are in general susceptible to image artifacts. Speckle is one such artifact that appears as randomly fluctuating bright and dark image pixels. Speckle arises from the manner in which ultrasound echoes from an object's surface are processed in an ultrasound imaging system. The reflections from a number of scatterers on the object surface are coherently added, allowing random fluctuations in the phase of these reflections to translate as an amplitude noise, thereby degrading image quality.

Another common ultrasound image artifact is shadowing. Shadowing occurs particularly in two-dimensional ultrasound images and results in darker image regions behind acoustically-dense image features. Shadowing arises from the reduced ultrasound signal propagating beyond such image features.

Image compounding refers to a group of techniques that are used in the ultrasound field to reduce such image artifacts by combining different images of a region of interest; see for instance the document entitled "Multi-Angle Compound Imaging" by Jespersen, S. K. et al., Ultrasonic Imaging, Vol. 20, pages 81 - 102, 1998. One form of image compounding termed "spatial compounding" involves the insonification of a region of interest at multiple incidence angles and combining the information into a single image. The combining of images from multiple angles reduces both shadowing, and also the variance of the speckle. Another form of image compounding termed "frequency compounding" that is described in more detail in a document "Phasing out speckle" by Gatenby, J. C. et al, Phys Med Biol. 1989 Nov, 34(11), pages 1683-9 involves the combining of image data that is generated at multiple insonification frequencies into a single image. The speckle patterns from each frequency are thereby averaged and the resulting speckle is likewise reduced. Frequency compounding has been used alone or in combination with spatial compounding.

A document entitled "A probabilistic framework for freehand 3D ultrasound reconstruction applied to catheter ablation guidance in the left atrium" by Koolwal, A. B., et al., Int. J. CARS (2009) 4:425-437 describes the use of an ICE catheter to collect 2D-ICE images of a left atrium phantom from multiple configurations and iteratively compound the acquired data into a 3D-ICE volume. Two methods for compounding overlapping ultrasound data are described: occupancy likelihood and response-grid compounding, which automatically classify voxels as "occupied" or "clear," and mitigate reconstruction artifacts caused by signal dropout.

Another document US20040044284A1 relates to methods that vary the elevation beam pattern during an imaging session. A user based or automatic search mode is provided where one or more elevation beam thicknesses are used, and then a diagnosis mode is provided where an optimal or narrow elevation beam thickness is used for continued imaging. 1.25, 1.5, 1.75 and 2D arrays are used to obtain frames of data responsive to the varied elevation beam pattern.

Document US2013131499 discloses compounding via multi-angle imaging for an ICE catheter. It teaches that a flexible assembly of ultrasound transducer elements is provided at the distal end of the catheter and an optical shape fiber arrangement is used for accurate determination of the position of one or more transducers relative to one another.

Another document "Spatial compounding for 2D and 3D volume ICE, based on catheter pose tracking from fluoroscopy" by Ralovich, K., et al, Prior Art Publishing, 2014, XP040638683, discloses an ICE catheter equipped with a radio-opaque markers that is tracked with a C-arm co-ordinate system. The tracked position of the catheter is used to determine the spatial relationship between different ICE imaging frames. It allows the registration of ICE volumes from different time points.

Another document US5876345A relates to an ultrasonic catheter having at least two ultrasonic arrays.

Another document US20070167823A1 relates to an imaging catheter assembly and method for use in volumetric ultrasound imaging and catheter-guided procedures. The imaging catheter assembly comprises a transducer array for acquiring image data at a given image plane and a motion controller coupled to the transducer array for translating the transducer array along a direction perpendicular to a direction of the image plane in order to image a three-dimensional (3D) volume.

In spite of these developments there remains room to improve ICE images.

### SUMMARY OF THE INVENTION

The present invention is defined in the independent claims and seeks to reduce speckle in ICE imaging procedures.

Further advantages from the described invention will also be apparent to the skilled person. Thereto an ultrasound image compounding method, a computer program product, an ICE catheter and an ultrasound imaging arrangement are presented. An ultrasound image compounding method suitable for use with an ICE catheter that includes a first ultrasound transceiver array and a second ultrasound transceiver array in which the first ultrasound transceiver array and the second ultrasound transceiver array are axially separated along a length of the ICE catheter is described.

The method includes the steps of: i) receiving, from the first ultrasound transceiver array, first array data corresponding to ultrasound signals detected by the first ultrasound transducer array in response to an insonification of a region of interest by the first ultrasound transducer array at a first insonification angle; ii) receiving, from the second ultrasound transceiver array, second array data corresponding to ultrasound signals detected by the second ultrasound transducer array in response to an insonification of the region of interest by the second ultrasound transducer array at a second insonification angle; and iii) generating, based on the first array data and the second array data, a compound image corresponding to the region of interest.

In so doing, at least some of the first array data and at least some of the second array data both correspond to the same region of interest; i.e. they correspond to an overlapping region. By so combining the data generated by the two axially-separated transducer arrays, the resulting compound image, i.e. a combined image that has been generated by insonification of the same region of interest at multiple insonification angles, has reduced speckle in this region of interest. This is because the speckle patterns from each array, being based on detected ultrasound signals from two insonification angles of the region of interest, are averaged in the compound image, i.e. combined image, which reduces the variance of the speckle. Moreover the two different insonification angles of the region of interest result in less shadowing behind acoustically-dense image features as compared to an ICE catheter that uses only a single viewing angle or a single array. The use of two axially-separated transducer arrays also advantageously increases the range of insonification angles that the region of interest can be subjected to, thereby further reducing shadowing. By reducing these image artifacts an improved ultrasound image is provided. In some implementations the separate arrays may be operated simultaneously. This reduces the time to scan the region of interest across the desired angular range and thereby reduces image blurring caused by intra-scan ICE catheter motion. In other implementations, when the two arrays are included on a flexible catheter, a bend in the catheter between the two arrays can be used to further reduce shadowing since this also increases the range of insonification angles that the region of interest can be subjected to.

In accordance with one aspect the first ultrasound transceiver array and the second ultrasound transceiver array have a mutual spatial arrangement. In this aspect the step of generating the compound image is based further on the mutual spatial arrangement.

The mutual spatial arrangement aspect includes the steps of receiving, from the first ultrasound transceiver array, first array tracking data corresponding to ultrasound signals detected by the first ultrasound transducer array in response to ultrasound signals emitted by the second ultrasound transducer array; or receiving, from the second ultrasound transceiver array, second array tracking data corresponding to ultrasound signals detected by the second ultrasound transducer array in response to ultrasound signals emitted by the first ultrasound transducer array; and determining the mutual spatial arrangement based on the first array tracking data or the second array tracking data correspondingly. In so doing, an ultrasound transceiver array's tracking data is provided by ultrasound signals that are emitted by the other of the two ultrasound transceiver arrays. The provision of tracking data in this manner, i.e. using ultrasound signals, provides a simple means of determining the mutual spatial arrangement because the existing ultrasound transceiver arrays are used. The mutual spatial arrangement may be determined by computing a distance between the two ultrasound transceiver arrays, the distance being computed based on a time of flight of the ultrasound signals. In one implementation the ultrasound signals may be dedicated tracking signals that are specifically directed towards the opposite ultrasound transceiver array. In another implementation the ultrasound signals may be stray ultrasound signals emitted by the other ultrasound transceiver array, for example a sidelobe of the opposite ultrasound transceiver array's insonification of the region of interest. In another implementation the ultrasound signals emitted by each ultrasound transducer array in the mutual spatial arrangement aspect may form a hemispherical wave front that radiates outwardly. In this last implementation a receiving ultrasound transducer array's tracking data may correspond to time of flight data indicative of a distance between the transmitting array and each of a plurality of array elements of the receiving ultrasound transceiver array. The mutual spatial arrangement may subsequently be determined based on the receiving ultrasound transducer array's tracking data by triangulating the position of each of the plurality of array elements of the receiving ultrasound transceiver array respective the transmitting array.

According to another example not falling within the scope of the invention, the mutual spatial arrangement aspect may include the use of data from a bend sensor or a bend actuator on the ICE catheter, the bend sensor or actuator being configured to provide bend data indicative of a bend of the catheter between the first ultrasound transceiver array and the second ultrasound transceiver array. In this aspect the method further includes the step of receiving the bend data and determining the mutual spatial arrangement based on a model configured to predict the mutual spatial arrangement based on the bend data. Advantageously this aspect provides a reliable compound image because the mutual positions of the two ultrasound transducer arrays are accurately determined.

According to a further example also not falling within the scope of the invention, the mutual spatial arrangement aspect may include the use of a first ultrasound transceiver array that is a two-dimensional array for generating a volumetric ultrasound image. In this aspect the mutual spatial arrangement is determined by: reconstructing a volumetric ultrasound image based on the first array data, the volumetric ultrasound image comprising a plurality of two-dimensional image slices; reconstructing a planar ultrasound image based on the second array data; and matching one of the plurality of two-dimensional image slices to the planar ultrasound image based on at least one image feature in the region of interest. Advantageously this aspect does not require additional sensors to determine the mutual spatial arrangement.

In accordance with another aspect an ICE catheter for use with any of the aforementioned methods is presented. The ICE catheter includes a first ultrasound transceiver array and a second ultrasound transceiver array in which the first ultrasound transceiver array and the second ultrasound transceiver array are axially separated along the length of the ICE catheter. The first ultrasound transceiver array and the second ultrasound transceiver array each include an array surface configured to transmit and to receive ultrasound signals. Preferably the array surface of the first ultrasound transceiver array and the array surface of the second ultrasound transceiver array are mutually parallel in a rotational direction about the length axis, or more specifically: mutually parallel in a direction that is tangential to the circumference of a virtual circle that is formed by rotating a point perpendicularly about the length axis. This aspect helps to improve the size of the region of interest, i.e. the overlapping insonified region. Optionally the ICE catheter may include a bend sensor or a bend actuator.

In accordance with another aspect an ultrasound imaging arrangement is presented. The ultrasound imaging arrangement includes an ICE catheter including a first ultrasound transceiver array and a second ultrasound transceiver array. The first ultrasound transceiver array and the second ultrasound transceiver array are axially separated along the length of the ICE catheter. The ultrasound imaging arrangement also includes a processor configured to execute any of the aforementioned methods.

Further aspects are described with reference to the appended claims.

### BRIEF DESCRIPTION OF THE DRAWINGS

Fig. 1 illustrates a prior art form of spatial compounding in which an ultrasound transducer array 110 insonifies an acoustically dense object 111 from multiple directions.
Fig. 2 illustrates a first embodiment of the invention in which an ICE catheter 220 includes a first ultrasound transceiver array 210₁ and a second ultrasound transceiver array 210₂.
Fig. 3 illustrates a method that may be used with the ICE catheter of Fig. 1.
Fig. 4 illustrates an ICE catheter 220 in which the mutual spatial arrangement of first ultrasound transceiver array 210₁ and second ultrasound transceiver array 210₂ is determined from ultrasound signals detected by an ultrasound transducer array in response to ultrasound signals emitted by the other ultrasound transducer array.
Fig. 5 illustrates an embodiment of an ICE catheter 220 that includes a bend sensor 215, not falling within the scope of the invention.
Fig. 6 illustrates an ultrasound imaging arrangement 600 that includes ICE catheter 220 and a processor 622.

### DETAILED DESCRIPTION OF EMBODIMENTS

In order to illustrate the principles of the present invention an ultrasound image compounding method is described with particular reference to an ICE catheter that is used to image cardiac structures. It is however to be appreciated that the ultrasound image compounding method also finds application with an ICE catheter that is used to image interventional devices such as an ablation catheter, a lasso catheter used for electrophysiological mapping of cardiac structures, and so forth. Moreover an ICE catheter is described that includes a first and a second ultrasound transducer array. The relative positions of the arrays, with the first ultrasound transducer array illustrated as being at the distal end of the catheter should not be interpreted as limiting. For example the relative positions of these arrays may be reversed such that the second ultrasound transducer array is at the distal end, or indeed the arrays may be positioned at other positions along the length of the ICE catheter than the distal end.

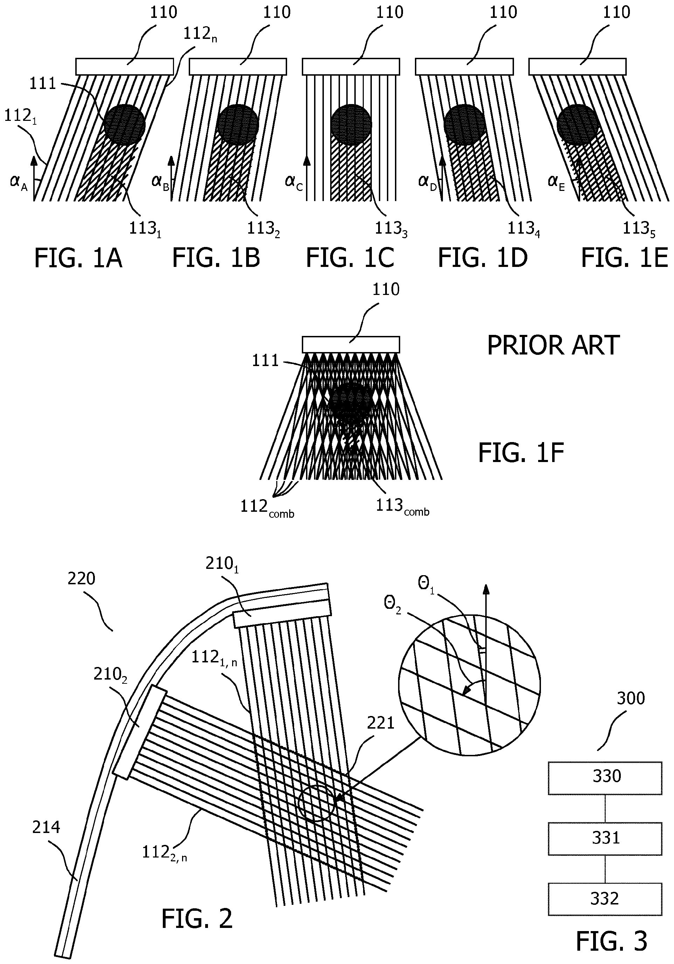

ICE is widely used during interventional cardiac procedures to visualize anatomical features such as the atrial septum, the aortic valve, pulmonary veins and so forth. As with other conventional ultrasound imaging systems, ICE suffers from two common image artifacts: speckle and shadow. One technique that is used in the ultrasound field to mitigate these artifacts is image compounding. Spatial compounding is a particular form of image compounding that involves the insonification of a region of interest at several incidence angles and combining the information into a single image. See for instance document "Multi-Angle Compound Imaging" by Jespersen, S. K. et al., Ultrasonic Imaging, Vol. 20, pages 81 - 102, 1998. In this regard, Fig. 1 illustrates a prior art form of spatial compounding in which an ultrasound transducer array 110 insonifies an acoustically dense object 111 from multiple directions. In the series of figures 1A..1E, image scan lines 112_{i=1..n} that insonify acoustically dense object 111 from each of several different angles α_{A..E} are generated using beam steering techniques by applying relative delays to the ultrasound signals emitted by and detected by the plurality of array elements that form ultrasound transducer array 110. By adjusting the insonification angle with respect to acoustically dense object 111, a different acoustic shadow 113_{i=1..n} is generated at each insonification angle α. The acoustic shadow is a region of lower intensity ultrasound signals that results from the blocking of ultrasound signals by acoustically dense object 111. In an ultrasound B-mode image, image features in this region tend to appear darker than would be expected owing to the reduced intensity ultrasound signals in this region. In the prior art technique of spatial compounding a combined image is generated by performing a weighted sum of the ultrasound image data, i.e. intensity, obtained at corresponding positions in the region of interest from each insonification angle. As illustrated in Fig. 1F, the combined effect of all image scan lines 112_{comb} is to reduce the combined shadow 113_{Comb}. A secondary effect of this weighting is a reduction in speckle. This results from the averaging of the speckle patterns from each angle α, wherein the averaging reduces the variance of the speckle.

Frequency compounding is another particular form of image compounding. Frequency compounding involves the insonification of the object at several frequencies and combining the information into a single image. See for instance the document "Phasing out speckle" by Gatenby, J. C., et al, Phys. Med. Biol., 1989, Vol. 34, No. 11, 1683 - 1689.

With reference to Fig. 1, the combination of frequency compounding and spatial compounding additionally involves, prior to generating the combined image: band pass filtering, at each of a plurality of central frequencies, the temporal ultrasound signals corresponding to each of the plurality of image scan lines 112_{i=1..n}, and computing for each image scan line, an weighted average of the band pass filtered ultrasound signals. Consequent to the weighting of the contributions from each frequency in the combined image, frequency compounding may further reduce speckle.

Fig. 2 illustrates a first embodiment of the invention in which an ICE catheter 220 includes a first ultrasound transceiver array 210₁ and a second ultrasound transceiver array 210₂. ICE catheter 220 includes a length axis 214. First ultrasound transceiver array 210₁ and second ultrasound transceiver array 210₂ are axially separated along the length of ICE catheter 220. The first ultrasound transceiver array and the second ultrasound transceiver array each include an array surface configured to transmit and to receive ultrasound signals. Preferably the array surface of the first ultrasound transceiver array and the array surface of the second ultrasound transceiver array are mutually parallel in a rotational direction about length axis 214, or more specifically: mutually parallel in a direction that is tangential to the circumference of a virtual circle that is formed by rotating a point perpendicularly about length axis 214. This helps to improve the size of region of interest 221, i.e. the overlapping insonified region. Fig. 3 illustrates a method that may be used with the ICE catheter of Fig. 2. With reference to Fig. 3 and Fig. 2, the method includes the steps of: receiving 330, from first ultrasound transceiver array 210₁, first array data corresponding to ultrasound signals detected by first ultrasound transducer array 210₁ in response to an insonification of region of interest 221 by first ultrasound transducer array 210₁ at first insonification angle θ₁; receiving 331, from second ultrasound transceiver array 210₂, second array data corresponding to ultrasound signals detected by second ultrasound transducer array 210₂ in response to an insonification of region of interest 221 by second ultrasound transducer array 210₂ at second insonification angle θ₂; and generating 332, based on the first array data and the second array data, a compound image corresponding to region of interest 221.

By so combining the data generated by axially-separated transducer arrays 210₁, 210₂, the resulting spatially-compounded image has reduced speckle. The speckle patterns from each array are averaged in the compound image, thereby reducing the variance of the speckle. Moreover the different insonification angles of the region of interest result in less shadowing behind acoustically-dense image features as compared to an ICE catheter that uses a single viewing angle or a single array. By reducing these image artifacts an improved ultrasound image is provided. The use of axially-separated transducer arrays 210₁, 210₂ advantageously increases the range of insonification angles θ₁, θ₂ that region of interest 221 can be subjected to, thereby further reducing shadowing. Moreover, the separate arrays 210₁, 210₂ may be operated simultaneously. This reduces the time to scan region of interest 221 across the desired angular range and thereby reduces image blurring caused by intra-scan motion of ICE catheter 220. When a flexible catheter is used for ICE catheter 220 a bend in ICE catheter 220 between the two arrays 210₁, 210₂ can be used to further reduce shadowing since this bend further increases the range of insonification angles that the region of interest can be subjected to. Such a flexible catheter is also termed a steerable catheter and may include a control unit configured to provide a desired bend. This facilitates e.g. the steering of the catheter around chambers in the heart.

Whilst an idealized array of parallel image scan lines 112_{1,n} and 112_{2,n} are illustrated in Fig. 2, it is to be appreciated that alternative beam patterns may be used. Such beam patterns may exemplarily include beam patterns that have a beam waist, beams that are generated using a subset array elements of the ultrasound transducer arrays, or beams that are steered using known beamforming techniques in directions other than the perpendicular directions with respect to arrays 210₁, 210₂ that are illustrated in Fig. 2. Clearly such alternative beams must, as illustrated in Fig. 2, likewise overlap at region of interest 221.

ICE catheter 220 in Fig. 2 is illustrated as including a bend between ultrasound transducer arrays 210₁, 210₂, however other shaped catheters including for example a straight catheter or a bendable or steerable catheter may alternatively be used.

Ultrasound transducer arrays 210₁, 210₂ may be one-dimensional or two-dimensional arrays, each including a plurality of array elements. Thereto the arrays may, correspondingly be used to generate and detect ultrasound signals corresponding to either a planar or a volumetric, i.e. 3D ultrasound images. Moreover, more than two ultrasound transducer arrays may be included on catheter 220 in the same manner. In one embodiment described later, one of the arrays is a two-dimensional array and another of the arrays is either a one- or a two-dimensional array. The array elements may include for example piezoelectric material or may be membrane-based i.e. Capacitive Micro machined Ultrasonic Transducers, a technology referred-to as CMUT.

In accordance with one implementation the method step of generating 332 includes: weighting the first array data and the second array data at corresponding positions in the region of interest 221; and summing the weighted first array data and the weighted second array data at the corresponding positions to provide the compound image. In some instances equal weighting of the first and second array data may be used. Differing weighting values may alternatively be used.

In accordance with one implementation the method step of generating 332 includes: reconstructing a first ultrasound image based on the first array data, the first ultrasound image including first ultrasound image intensity data; reconstructing a second ultrasound image based on the second array data, the second ultrasound image including second ultrasound image intensity data; weighting the first ultrasound image intensity data and the second ultrasound image intensity data at corresponding positions in the region of interest 221; and summing the weighted first ultrasound image intensity data and the weighted second ultrasound image intensity data at the corresponding positions to provide the compound image. One of many known ultrasound image reconstruction techniques may be used, for example to reconstruct brightness, or B-mode ultrasound images that include the ultrasound image intensity data. Although other weighting factors may be used, preferably a weighting is used in which each of the two ultrasound images contribute equally to corresponding positions in the compound image.

In accordance with another implementation the method step of generating 332 includes: comparing the first array data and the second array data at corresponding positions in region of interest 221 and selecting the largest value of said data at each corresponding position to provide the compound image. This form of image compounding offers improved boundary definition at the expense of somewhat lowered speckle reduction.

In accordance with another implementation, frequency compounding may be used in combination with the above-described techniques of spatial compounding. Thereto, in this implementation the first array data includes temporal ultrasound signals corresponding to each of a plurality of image scan lines at the first insonification angle 112_{1,n}; and the second array data includes temporal ultrasound signals corresponding to each of a plurality of image scan lines at the second insonification angle 112_{2,n}. Moreover, the step of generating a compound image corresponding to the region of interest 221 includes: band pass filtering, at each of a plurality of central frequencies, the temporal ultrasound signals corresponding to each of a plurality of image scan lines at the first insonification angle 112_{1,n} and computing for each image scan line, a weighted average of the band pass filtered ultrasound signals; band pass filtering, at each of a plurality of central frequencies, the temporal ultrasound signals corresponding to each of a plurality of image scan lines at the second insonification angle 112_{2,n} and computing for each image scan line, a weighted average of the band pass filtered ultrasound signals; and summing the weighted average of the band pass filtered ultrasound signals at the first insonification angle 112_{1,n} and the weighted average of the band pass filtered ultrasound signals at the second insonification angle 112_{2,n} at corresponding positions in the region of interest 221 to provide the compound image. In so doing, additional speckle reduction may be achieved.

In accordance with the invention, first ultrasound transceiver array 210₁ and second ultrasound transceiver array 210₂ have a mutual spatial arrangement. In this implementation the step of generating 332 is based further on the mutual spatial arrangement. In so doing, any changes in the mutual spatial arrangement can be accounted-for in the generated compound image.

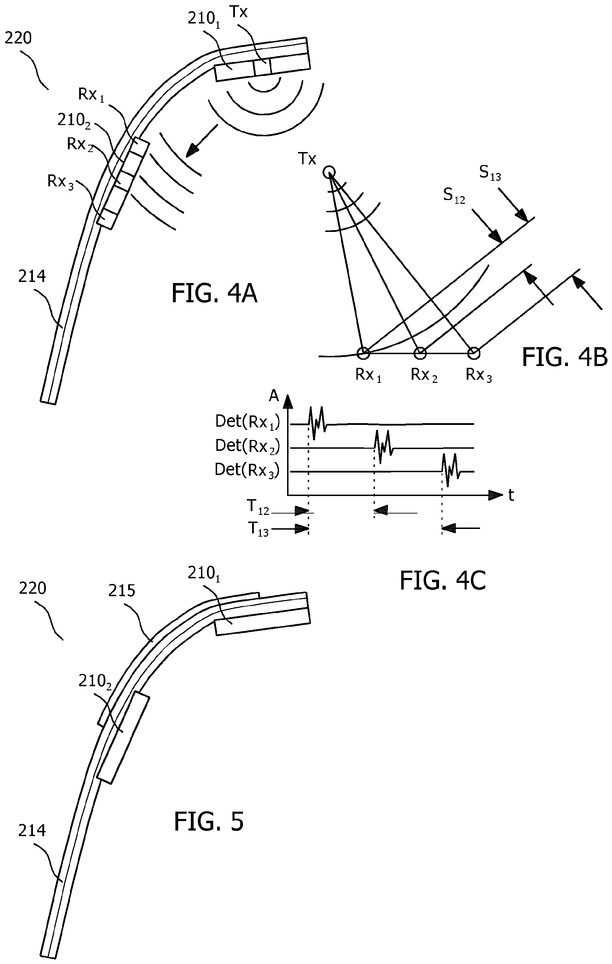

In the mutual spatial arrangement implementation, array tracking data is generated by one of the ultrasound transceiver arrays 210₁, 210₂. In this embodiment an ultrasound transceiver array's tracking data is provided by ultrasound signals that are emitted by the other of the two ultrasound transceiver arrays. Thereto, the method may include the steps of: receiving, from first ultrasound transceiver array 210₁, first array tracking data corresponding to ultrasound signals detected by the first ultrasound transducer array 210₁ in response to ultrasound signals emitted by the second ultrasound transducer array 210₂; or receiving, from the second ultrasound transceiver array 210₂, second array tracking data corresponding to ultrasound signals detected by the second ultrasound transducer array 210₂ in response to ultrasound signals emitted by the first ultrasound transducer array 210₁; and determining the mutual spatial arrangement based on the first array tracking data or the second array tracking data correspondingly. An ultrasound transducer array's emitted signals may be dedicated tracking pulses, or indeed stray ultrasound signals such as a sidelobe of those that are used by that ultrasound transducer array to insonify the region of interest. The latter has the benefit of simplifying the control of the ultrasound signals emitted by an array because it involves detecting the other ultrasound transducer array's stray imaging signals. Such sidelobes are typically present as a result of the beamforming techniques that are used to insonify region of interest 221 at the respective insonification angle θ₁, θ₂. The benefit of using dedicated tracking pulses is that these may advantageously be directed towards the expected positon of the other, receiving, array, thereby minimizing the emitted ultrasound power. One example implementation of this mutual spatial arrangement is illustrated in Fig. 4, which illustrates an embodiment of an ICE catheter 220 in which the mutual spatial arrangement of first ultrasound transceiver array 210₁ and second ultrasound transceiver array 210₂ is determined from ultrasound signals detected by an ultrasound transducer array in response to ultrasound signals emitted by the other ultrasound transducer array.

In Fig. 4A and Fig. 4B, first ultrasound transceiver array 210₁ operates as an emitter with a transmitter element Tx and second ultrasound transceiver array 210₂ operates as a detector with multiple detector elements Rx_{1..3}. As illustrated in Fig. 4B and Fig. 4C, ultrasound signals in the form of an ultrasound pulse emitted by transmitter Tx impinge upon detectors Rx_{1..3}. The pulse is detected by detectors Rx_{1..3} as illustrated by signals Det(Rx_{1..3}) and the times of flight of each of the ultrasound signals correspond to the distances between transmitter Tx and the corresponding receiver Rx_{1..3}. Differences T₁₂, T₁₃, in these times of flight corresponds to differences S₁₂, S₁₃ in these distances. The mutual spatial arrangement of first ultrasound transceiver array 210₁ and second ultrasound transceiver array 210₂ can subsequently be calculated via triangulation based on these distances. Clearly the same effect may also be achieved by reversing the functionality of first ultrasound transceiver array 210₁ and second ultrasound transceiver array 210₂ such that first ultrasound transceiver array 210₁ operates as a detector. Although triangulation based on three distances is illustrated in Fig 4, in some cases three distances are not required; for example when the bending of ICE catheter 220 is constrained such that it can only bend in one dimension. In this case a single distance measurement may suffice.

As illustrated in Fig. 4A, the ultrasound signals emitted by first ultrasound transducer array 210₁ may form a hemispherical wave front radiating outwardly with respect to the first ultrasound transducer array. Such a wave front may be beneficial when ICE catheter 220 has significant bending freedom owing to its relatively uniform power distribution in directions away from first ultrasound transducer array 210₁. Alternatively, beam steering techniques may be used to generate a more directed beam that is directed more specifically at the opposite transducer array in order to reduce the power required from the ultrasound signals. As an alternative to the dedicated ultrasound signals of this and the hemispherical wave front example, as mentioned above, stray ultrasound signals, i.e. a sidelobe of the other array's insonification of the region of interest may be detected in the same manner.

Fig. 5 illustrates an embodiment of an ICE catheter 220 not falling within the scope of the invention. The ICE catheter includes a bend sensor 215. Bend sensor 215 may be used to determine the mutual spatial arrangement of first ultrasound transceiver array 210₁ and second ultrasound transceiver array 210₂ as an alternative to the above-described ultrasound signals. Bend sensor 215 extends between first ultrasound transceiver array 210₁ and second ultrasound transceiver array 210₂. Bend sensor 215 may for example be a strain gauge such as a fiber Bragg grating, or a capacitive position sensor or another type of bend sensor altogether. In so doing, bend sensor 215 provides bend data indicative of a bend of ICE catheter 220 between first ultrasound transceiver array 210₁ and second ultrasound transceiver array 210₂. The bend data may be used in combination with a model that describes the mutual spatial arrangement of first ultrasound transceiver array 210₁ and second ultrasound transceiver array 210₂ based on the bend data, to determine the mutual spatial arrangement. In another configuration that is not illustrated, ICE catheter 220 may include a bend actuator configured to provide a desired bend and corresponding bend data indicative of a bend of ICE catheter 220 between first ultrasound transceiver array 210₁ and second ultrasound transceiver array 210₂. In the same way, the bend data may be used in combination with a model that describes the mutual spatial arrangement of first ultrasound transceiver array 210₁ and second ultrasound transceiver array 210₂ based on the bend data, to determine the mutual spatial arrangement. A known type of actuator for use in such a steerable catheter uses wires that extend within the catheter, which when provided with a predetermined tension provide a desired catheter bend. Upon releasing the tension the catheter relaxes back to its original form. Such models may for example relate a predetermined bend or ICE catheter tip displacement to empirical strain measurements.

In another embodiment that is described with reference to Fig. 2, not falling within the scope of the invention, instead of using the above-described bend data and instead of using the above-described ultrasound signals to determine the mutual spatial arrangement of first ultrasound transceiver array 210₁ and second ultrasound transceiver array 210₂, the mutual spatial arrangement may be determined by matching ultrasound images generated by each ultrasound transceiver array. In this embodiment, first ultrasound transceiver array 210₁ is a two-dimensional array for generating a volumetric ultrasound image. The mutual spatial arrangement is determined by: reconstructing a volumetric ultrasound image based on the first array data, the volumetric ultrasound image comprising a plurality of two-dimensional image slices; reconstructing a planar ultrasound image based on the second array data; and matching one of the plurality of two-dimensional image slices to the planar ultrasound image based on at least one image feature in the region of interest. Various known image matching algorithms may be used to perform the matching. Scale-invariant feature transform, SIFT, is one suitable example. Rigid or non-rigid image transforms known from the medical image processing field may also be used. The correlation technique described in US patent 5,655,535, or the real-time image alignment procedure described in US patent 8,303,505 may alternatively be used. Clearly the same effect may be achieved by also making the second ultrasound transceiver array 210₁ a two-dimensional array for generating a volumetric ultrasound image. In this regard, both of the arrays 210₁, 210₂ may be two-dimensional arrays in which one of the arrays is configured to generate a volumetric image and the other is configured to generate a planar image.

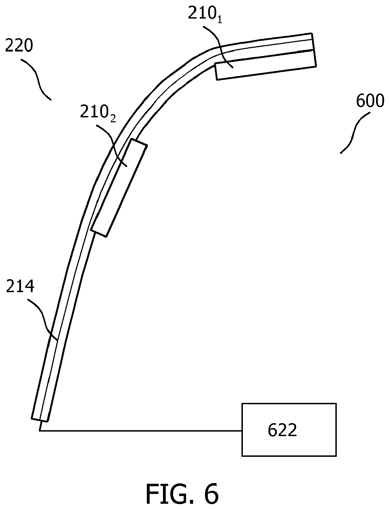

Any of the method steps presented herein, may be recorded in the form of instructions which when executed on a processor cause the processor to carry out such method steps. Thereto, Fig. 6 illustrates an ultrasound imaging arrangement 600 that includes ICE catheter 220 and a processor 622. Data transfer between ultrasound transducer arrays 210₁, 210₂ and processor 622 is illustrated by way of the link between these items. It is to be appreciated that either wired or wireless data communication is contemplated for this link. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor "DSP" hardware, read only memory "ROM" for storing software, random access memory "RAM", non-volatile storage, etc. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray^{™} and DVD.

In summary, an ultrasound image compounding method has been described that is suitable for use with an ICE catheter that includes a first ultrasound transceiver array and a second ultrasound transceiver array, the first ultrasound transceiver array and the second ultrasound transceiver array being axially separated along a length of the ICE catheter. In the method, first array data corresponding to ultrasound signals detected by the first ultrasound transducer array in response to an insonification of a region of interest by the first ultrasound transducer array at a first insonification angle; and second array data corresponding to ultrasound signals detected by the second ultrasound transducer array in response to an insonification of the region of interest by the second ultrasound transducer array at a second insonification angle; are received. A compound image corresponding to the region of interest is generated based on the first array data and the second array data.

Various embodiments and implementations have been described in the above and it is noted that these may be combined to achieve further advantageous effects, however the scope of the invention is defined by the appended claims.

## Claims

1. Computer program product comprising instructions which when executed on a processor are configured to cause the processor to carry out an ultrasound image compounding method (300) for use with an Intra Cardiac Echography (ICE) catheter (220) including a first ultrasound transceiver array (210₁) and a second ultrasound transceiver array (210₂) in which the first ultrasound transceiver array (210₁) and the second ultrasound transceiver array (210₂) are axially separated along a length axis (214) of the ICE catheter (220) such that the first ultrasound transceiver array (210₁) and the second ultrasound transceiver array (210₂) have a mutual spatial arrangement; the method (300) comprising the steps of:
receiving (330), from the first ultrasound transceiver array (210₁), first array data corresponding to ultrasound signals detected by the first ultrasound transducer array (210₁) in response to an insonification of a region of interest (221) by the first ultrasound transducer array (210₁) at a first insonification angle (θ₁);
receiving (331), from the second ultrasound transceiver array (210₂), second array data corresponding to ultrasound signals detected by the second ultrasound transducer array (210₂) in response to an insonification of the region of interest (221) by the second ultrasound transducer array (210₂) at a second insonification angle (θ₂), different from the first insonification angle;
generating (332), based on the first array data and the second array data and the mutual spatial arrangement, a compound image corresponding to the region of interest (221); the method (300) further comprising the steps of:
receiving, from the first ultrasound transceiver array (210₁), first array tracking data corresponding to ultrasound signals detected by the first ultrasound transducer array (210₁) in response to ultrasound signals emitted by the second ultrasound transducer array (210₂); or
receiving, from the second ultrasound transceiver array (210₂), second array tracking data corresponding to ultrasound signals detected by the second ultrasound transducer array (210₂) in response to ultrasound signals emitted by the first ultrasound transducer array (210₁); and
determining the mutual spatial arrangement based on the first array tracking data or the second array tracking data correspondingly.

2. The computer program product according to claim 1 wherein the generating a compound image (332) includes:
weighting the first array data and the second array data at corresponding positions in the region of interest (221); and
summing the weighted first array data and the weighted second array data at the corresponding positions to provide the compound image.

3. The computer program product according to claim 1 wherein the generating a compound image (332) includes:
reconstructing a first ultrasound image based on the first array data, the first ultrasound image including first ultrasound image intensity data;
reconstructing a second ultrasound image based on the second array data, the second ultrasound image including second ultrasound image intensity data;
weighting the first ultrasound image intensity data and the second ultrasound image intensity data at corresponding positions in the region of interest (221); and
summing the weighted first ultrasound image intensity data and the weighted second ultrasound image intensity data at the corresponding positions to provide the compound image.

4. The computer program product according to claim 1 wherein the generating a compound image (332) includes:
comparing the first array data and the second array data at corresponding positions in the region of interest (221) and selecting the largest value of said data at each corresponding position to provide the compound image.

5. The computer program product according to any one of claims 1 to 4 wherein:
the first array data includes temporal ultrasound signals corresponding to each of a plurality of image scan lines at the first insonification angle (112_{1,n});
the second array data includes temporal ultrasound signals corresponding to each of a plurality of image scan lines at the second insonification angle (112_{2,n});
wherein the generating a compound image (332) includes:
band pass filtering, at each of a plurality of central frequencies, the temporal ultrasound signals corresponding to each of a plurality of image scan lines at the first insonification angle (112_{1,n}) and computing for each image scan line, a weighted average of the band pass filtered ultrasound signals;
band pass filtering, at each of a plurality of central frequencies, the temporal ultrasound signals corresponding to each of a plurality of image scan lines at the second insonification angle (112_{2,n}) and computing for each image scan line, a weighted average of the band pass filtered ultrasound signals; and
summing the weighted average of the band pass filtered ultrasound signals at the first insonification angle (112_{1,n}) and the weighted average of the band pass filtered ultrasound signals at the second insonification angle (112_{2,n}) at corresponding positions in the region of interest (221) to provide the compound image.

6. The computer program product according to claim 1 wherein the ultrasound signals emitted by the second ultrasound transducer array (210₂) correspond to at least one sidelobe of the insonification of the region of interest (221) by the second ultrasound transducer array (210₂) at the second insonification angle (θ₂); or wherein the ultrasound signals emitted by the first ultrasound transducer array (210₁) correspond to at least one sidelobe of the insonification of the region of interest (221) by the first ultrasound transducer array at the first insonification angle (θ₁), correspondingly.

7. The computer program product according to claims 1 or 6 wherein the step of determining the mutual spatial arrangement based on the corresponding first array tracking data or the corresponding second array tracking data, comprises:
for the first array tracking data, computing at least one distance between the first ultrasound transceiver array (210₁) and the second ultrasound transceiver array (210₂) based on a time of flight of the ultrasound signals detected by the first ultrasound transducer array (210₁) in response to ultrasound signals emitted by the second ultrasound transducer array (210₂), and
comprises for the second array tracking data, computing at least one distance between the first ultrasound transceiver array (210₁) and the second ultrasound transceiver array (210₂) based on a time of flight of the ultrasound signals detected by the second ultrasound transducer array (210₂) in response to ultrasound signals emitted by the first ultrasound transducer array (210₁).

8. The computer program product according to claim 1 wherein:
the ultrasound signals emitted by the second ultrasound transducer array (210₂) form a hemispherical wave front radiating outwardly with respect to the second ultrasound transducer array (210₂), wherein the first array tracking data corresponds to time of flight data indicative of a distance between the second ultrasound transducer array (210₂) and each of a plurality of array elements of the first ultrasound transceiver array (210₁), and wherein the mutual spatial arrangement is determined based on the first array tracking data by triangulating the position of each of the plurality of array elements of the first ultrasound transceiver array (210₁) respective the second ultrasound transducer array (210₂); or
wherein the ultrasound signals emitted by the first ultrasound transducer array (Tx, 210₁) form a hemispherical wave front radiating outwardly with respect to the first ultrasound transducer array (Tx, 210₁), wherein the second array tracking data corresponds to time of flight data indicative of a distance between the first ultrasound transducer array (Tx, 210₁) and each of a plurality of array elements of the second ultrasound transceiver array (Rx_{1..3}, 210₂), and wherein the mutual spatial arrangement is determined based on the second array tracking data by triangulating the position of each of the plurality of array elements of the second ultrasound transceiver array (Rx_{1..3}, 210₂) respective the first ultrasound transducer array (Tx, 210₁), correspondingly.

9. Ultrasound imaging arrangement (600) comprising:
an Intra Cardiac Echography (ICE) catheter (220) comprising a first ultrasound transceiver array (210i) and a second ultrasound transceiver array (210₂) in which the first ultrasound transceiver array (210₁) and the second ultrasound transceiver array (210₂) are axially separated along a length axis (214) of the ICE catheter (220) such that the first ultrasound transceiver array (210₁) and the second ultrasound transceiver array (210₂) have a mutual spatial arrangement; and
a processor (622) configured to execute the method steps of:
receiving (330), from the first ultrasound transceiver array (210₁), first array data corresponding to ultrasound signals detected by the first ultrasound transducer array (210₁) in response to an insonification of a region of interest (221) by the first ultrasound transducer array (210₁) at a first insonification angle (θ₁);
receiving (331), from the second ultrasound transceiver array (210₂), second array data corresponding to ultrasound signals detected by the second ultrasound transducer array (210₂) in response to an insonification of the region of interest (221) by the second ultrasound transducer array (210₂) at a second insonification angle (θ₂), different from the first insonification angle;
generating (332), based on the first array data and the second array data and the mutual spatial arrangement, a compound image corresponding to the region of interest (221); the method (300) further comprising the steps of:
receiving, from the first ultrasound transceiver array (210₁), first array tracking data corresponding to ultrasound signals detected by the first ultrasound transducer array (210₁) in response to ultrasound signals emitted by the second ultrasound transducer array (210₂); or
receiving, from the second ultrasound transceiver array (210₂), second array tracking data corresponding to ultrasound signals detected by the second ultrasound transducer array (210₂) in response to ultrasound signals emitted by the first ultrasound transducer array (210₁); and
determining the mutual spatial arrangement based on the first array tracking data or the second array tracking data correspondingly.

## Patentansprüche

1. Computerprogrammprodukt mit Anweisungen, die bei Ausführung auf einem Prozessor konfiguriert sind, um den Prozessor zu veranlassen, ein Ultraschallbild-Zusammensetzungsverfahren (300) zur Verwendung mit einem intrakardialen Echographie-(ICE)-Katheter (220) auszuführen, einschließlich eines ersten UltraschallWandler-Arrays (210₁) und eines zweiten Ultraschallwandler-Arrays (210₂), wobei das erste Ultraschallwandler-Array (210₁) und das zweite Ultraschallwandler-Array (210₂) sich axial getrennt entlang einer Längsachse (214) des ICE-Katheters (220) befinden, so dass das erste Ultraschallwandler-Array (210₁) und das zweite Ultraschallwandler-Array (210₂) eine gegenseitige räumliche Anordnung aufweisen; wobei das Verfahren (300) die folgenden Schritte umfasst:
Empfangen (330), vom ersten Ultraschallwandler-Array (210₁), erster Array-Daten, die Ultraschallsignalen entsprechen, die vom ersten Ultraschallwandler-Array (210₁) als Reaktion auf eine Beschallung eines Bereichs von Interesse (221) erfasst werden, durch das erste Ultraschallwandler-Array (210₁) in einem ersten Beschallungswinkel (θ₁);
Empfangen (331), durch das zweite Ultraschallwandler-Array (210₂), von Daten des zweiten Arrays, die Ultraschallsignalen entsprechen, die vom zweiten Ultraschallwandler-Array (210₂) als Reaktion auf eine Beschallung des interessierenden Bereichs (221) durch das zweite Ultraschallwandler-Array (210₂) erfasst werden, in einem zweiten Beschallungswinkel (θ₂), der sich vom ersten Beschallungswinkel unterscheidet;
Erzeugen (332), basierend auf den ersten Array-Daten und den zweiten Array-Daten und der gegenseitigen räumlichen Anordnung, eines zusammengesetzten Bildes, das dem interessierenden Bereich (221) entspricht; wobei das Verfahren (300) ferner die folgenden Schritte umfasst:
Empfangen, vom ersten Ultraschallwandler-Array (210₁), von Verfolgungs-Daten des ersten Arrays, die Ultraschallsignalen entsprechen, die vom ersten Ultraschallwandler-Array (210₁) als Reaktion auf vom zweiten Ultraschallwandler-Array (210₂) emittierte Ultraschallsignale erfasst werden; oder
Empfangen, durch das zweite Ultraschallwandler-Array (210₂), von Verfolgungsdaten des zweiten Arrays, die Ultraschallsignalen entsprechen, die vom zweiten Ultraschall-Koppler-Array (210₂) als Reaktion auf Ultraschallsignale erkannt werden, die vom ersten Ultraschall-Koppler-Array (210₁) ausgesendet werden; und
Bestimmen der gegenseitigen räumlichen Anordnung basierend auf den Verfolgungsdaten des ersten Arrays oder den Verfolgungsdaten des zweiten Arrays, entsprechend.

2. Computerprogrammprodukt nach Anspruch 1, wobei das Erzeugen eines zusammengesetzten Bildes (332) umfasst:
Gewichten der ersten Array-Daten und der zweiten ArrayDaten an entsprechenden Positionen in dem interessierenden Bereich (221); und
Summieren der gewichteten ersten Array-Daten und der gewichteten zweiten Array-Daten an den entsprechenden Positionen, um das zusammengesetzte Bild bereitzustellen.

3. Computerprogrammprodukt nach Anspruch 1, wobei das Erzeugen eines zusammengesetzten Bildes (332) umfasst:
Rekonstruieren eines ersten Ultraschallbildes basierend auf den ersten Array-Daten, wobei das erste Ultraschallbild erste Ultraschallbild-Intensitätsdaten enthält;
Rekonstruieren eines zweiten Ultraschallbildes basierend auf den zweiten Array-Daten, wobei das zweite Ultraschallbild zweite Ultraschallbild-Intensitätsdaten enthält;
Gewichten der ersten Ultraschallbild-Intensitätsdaten und der zweiten Ultraschallbild-Intensitätsdaten an entsprechenden Positionen in dem interessierenden Bereich (221); und
Summieren der gewichteten ersten Ultraschallbild-Intensitätsdaten und der gewichteten zweiten Ultraschallbild-Intensitätsdaten an den entsprechenden Positionen, um das zusammengesetzte Bild bereitzustellen.

4. Computerprogrammprodukt nach Anspruch 1, wobei das Erzeugen eines zusammengesetzten Bildes (332) umfasst:
Vergleichen der ersten Array-Daten und der zweiten ArrayDaten an entsprechenden Positionen in dem interessierenden Bereich (221) und Auswählen des größten Wertes der besagten Daten an den entsprechenden Positionen, um das zusammengesetzte Bild bereitzustellen.

5. Computerprogrammprodukt nach einem der Ansprüche 1 bis 4, wobei die ersten Arraydaten zeitliche Ultraschallsignale enthalten, die jeweils einer Vielzahl von Bildabtastlinien beim ersten Beschallungswinkel (112_{1,n}) entsprechen;
Die Daten des zweiten Arrays umfassen zeitliche Ultraschallsignale, die jeweils einer Vielzahl von Bildabtastlinien beim zweiten Beschallungswinkel (112_{2,1}) entsprechen;
wobei das Erzeugen eines zusammengesetzten Bildes (332) ein Bandpassfiltern, bei jeder der Mehrzahl von zentralen Frequenzen, der zeitlichen Ultraschallsignale enthält, die jeweils einer Mehrzahl von Abtastlinien beim ersten Beschallungswinkel (112_{1,n}) entsprechen, und ein Berechnen, für jede der Abtastlinien des Bildes, eines gewichteten Mittelwerts der bandpassgefilterten Ultraschallsignale;
Bandpassfiltern, bei jeder der Mehrzahl von zentralen Frequenzen, der zeitlichen Ultraschallsignale, die jeweils einer Vielzahl von Bildabtastzeilen beim zweiten Beschallungswinkel (112_{2,n}) entsprechen, und Berechnen eines gewichteten Mittelwerts der bandpassgefilterte Ultraschallsignale für jede Bildabtastzeile; und
Summieren des gewichteten Mittelwerts der bandpassgefilterten Ultraschallsignale beim ersten Beschallungswinkel (112_{1,n}) und des gewichteten Mittelwerts der bandpassgefilterten Ultraschallsignale beim zweiten Beschallungswinkel (112_{2,n}) an entsprechenden Positionen im Bereich von Interesse (221), um das zusammengesetzte Bild bereitzustellen.

6. Computerprogrammprodukt nach Anspruch 1, wobei die vom zweiten Ultraschallwandler-Array (210₂) emittierten Ultraschallsignale mindestens einer Nebenkeule der Beschallung des interessierenden Bereichs (221) durch das zweite Ultraschallwandler-Array (210₂) beim zweiten Beschallungswinkel (θ₂) entsprechen; oder wobei die vom ersten Ultraschallwandler-Array (210₁) ausgesendeten Ultraschallsignale mindestens einer Nebenkeule der Beschallung des interessierenden Bereichs (221) durch das erste Ultraschallwandler-Array beim ersten Beschallungswinkel (θ₁) entsprechen, entsprechend.

7. Computerprogrammprodukt nach Anspruch 1 oder 6, wobei der Schritt des Bestimmens der gegenseitigen räumlichen Anordnung, basierend auf den entsprechenden ersten Array-Verfolgungsdaten oder den entsprechenden zweiten Array-Verfolgungsdaten, umfasst:
Für die ersten Array-Verfolgungsdaten, das Berechnen mindestens eines Abstands zwischen dem ersten Ultraschallwandler-Array (210₁) und dem zweiten Ultraschallwandler-Array (210₂), basierend auf einer Laufzeit der Ultraschallsignale, die vom ersten Ultraschall-Koppler-Array (210₁) als Reaktion auf Ultraschallsignale detektiert werden, die vom zweiten Ultraschall-Koppler-Array (210₂) emittiert werden, und
für die zweiten Array-Verfolgungsdaten, das Berechnen mindestens eines Abstands zwischen dem ersten Ultraschallwandler-Array (210₁) und dem zweiten Ultraschallwandler-Array (210₂), basierend auf einer Laufzeit der Ultraschallsignale, die vom zweiten Ultraschallwandler-Array (210₂) als Reaktion auf Ultraschallsignale erfasst werden, die vom ersten Ultraschallwandler-Array (210₁) emittiert werden.

8. Computerprogrammprodukt nach Anspruch 1, wobei die vom zweiten Ultraschallwandler-Array (210₂) emittierten Ultraschallsignale eine halbkugelförmige Wellenfront bilden, die in Bezug auf das zweite Ultraschallwandler-Array (210₂) nach außen strahlt, wobei die ersten Array-Verfolgungsdaten Laufzeitdaten entsprechen, die einen Abstand zwischen dem zweiten Ultraschallwandler-Array (210₂) und jedem einer Mehrzahl von Array-Elementen des ersten Ultraschallwandler-Arrays (210₁) anzeigen, und wobei die gegenseitige räumliche Anordnung auf der Grundlage der ersten Array-Verfolgungsdaten durch Triangulieren der Position jedes der Mehrzahl von Array-Elementen des ersten Ultraschallwandler-Arrays (210₁) bzw. des zweiten Ultraschallwandler-Arrays (210₂) bestimmt wird; oder wobei die vom ersten Ultraschallwandler-Array (Tx, 210₁) emittierten Ultraschallsignale eine halbkugelförmige Wellenfront bilden, die in Bezug auf das erste Ultraschallwandler-Array (Tx, 210₁) nach außen strahlt, wobei die Verfolgungsdaten des zweiten Arrays Laufzeitdaten entsprechen, die einen Abstand zwischen dem ersten Ultraschallwandler-Array (Tx, 210₁) und jedem einzelnen einer Vielzahl von Arrayelementen des zweiten Ultraschallwandler-Arrays (RX_{1..3}, 210₂) anzeigen, und wobei die gegenseitige räumliche Anordnung basierend auf den Verfolgungsdaten des zweiten Arrays durch Triangulieren der Position jedes der Mehrzahl von ArrayElementen des zweiten Ultraschallwandler-Arrays (RX_{1..3}, 210₂) bzw, des ersten Ultraschallwandler-Arrays (Tx, 210₁) bestimmt wird, entsprechend.

9. Ultraschall-Bildgebungsanordnung (600), umfassend:
einen intrakardialen Echographie-(ICE)-Katheter (220), der ein erstes Ultraschallwandler-Array (210₁) und ein zweites Ultraschallwandler-Array (210₂) umfasst, wobei das erste Ultraschallwandler-Array (210₁) und das zweite Ultraschallwandler-Array (210₂) entlang einer Längsachse (214) des ICE-Katheters (220) axial getrennt sind, so dass das erste Ultraschallwandler-Array (210₁) und das zweite Ultraschallwandler-Array (210₂) eine gegenseitige Anordnung im Raum haben; und
einen Prozessor (622), konfiguriert, um folgende Methodenschritte auszuführen:
Empfangen (330), vom ersten Ultraschallwandler-Array (210₁), erster Array-Daten, die Ultraschallsignalen entsprechen, die vom ersten Ultraschallwandler-Array (210₁) als Reaktion auf eine Beschallung eines Bereichs von Interesse (221) erfasst werden, durch das erste Ultraschallwandler-Array (210₁) in einem ersten Beschallungswinkel (θ₁);
Empfangen (331) durch das zweite Ultraschallwandler-Array (210₂) von Daten des zweiten Arrays, die Ultraschallsignalen entsprechen, die vom zweiten Ultraschallwandler-Array (210₂) als Reaktion auf eine Beschallung des interessierenden Bereichs (221) durch das zweite Ultraschallwandler-Array (210₂) erfasst werden, in einem zweiten Beschallungswinkel (θ₂), der sich vom ersten Beschallungswinkel unterscheidet;
Erzeugen (332), basierend auf den ersten Array-Daten und den zweiten Array-Daten und der gegenseitigen räumlichen Anordnung, eines zusammengesetzten Bildes, das dem interessierenden Bereich (221) entspricht; wobei das Verfahren (300) ferner die folgenden Schritte umfasst:
Empfangen, vom ersten Ultraschallwandler-Array (210₁), von Verfolgungsdaten des ersten Arrays, die Ultraschallsignalen entsprechen, die vom ersten Ultraschallwandler-Array (210₁) als Reaktion auf vom zweiten Ultraschallwandler-Array (210₂) emittierten Ultraschallsignale erfasst werden; oder
Empfangen, durch das zweite Ultraschallwandler-Array (210₂), von Verfolgungsdaten des zweiten Arrays, die Ultraschallsignalen entsprechen, die vom zweiten Ultraschall-Koppler-Array (210₂) als Reaktion auf Ultraschallsignale erkannt werden, die vom ersten Ultraschall-Koppler-Array (210₁) ausgesendet werden; und
Bestimmen der gegenseitigen räumlichen Anordnung, basierend auf den Verfolgungsdaten des ersten Arrays oder den Verfolgungsdaten des zweiten Arrays, entsprechend.

## Revendications

1. Produit de programme d'ordinateur comprenant des instructions qui, lorsqu'elles sont exécutées sur un processeur, sont configurées pour amener le processeur à exécuter un procédé de composition d'image ultrasonore (300) destiné à être utilisé avec un cathéter d'échographie intracardiaque (ICE) (220) comprenant un premier réseau d'émetteurs-récepteurs à ultrasons (210₁) et un second réseau d'émetteursrécepteurs à ultrasons (210₂) dans lequel le premier réseau d'émetteurs-récepteurs à ultrasons (210₁) et le second réseau d'émetteurs-récepteurs à ultrasons (210₂) sont séparés axialement le long d'un axe de longueur (214) du cathéter ICE (220) de sorte que le premier réseau d'émetteurs-récepteurs à ultrasons (210₁) et le second réseau d'émetteurs-récepteurs à ultrasons (210₂) ont un agencement spatial mutuel;
le procédé (300) comprenant les étapes consistant à: recevoir (330), à partir du premier réseau émetteur-récepteur à ultrasons (210₁), des premières données de réseau correspondant à des signaux ultrasonores détectés par le premier réseau transducteur à ultrasons (210₁) en réponse à une insonification d'une région d'intérêt (221) par le premier réseau transducteur à ultrasons (210₁) à un premier angle d'insonification (θ₁);
recevoir (331), à partir du second réseau émetteur-récepteur à ultrasons (210₂), des secondes données de réseau correspondant à des signaux ultrasonores détectés par le second réseau transducteur à ultrasons (210₂) en réponse à une insonification de la région d'intérêt (221) par le second réseau transducteur à ultrasons (210₂) à un second angle d'insonification (θ₂), différent du premier angle d'insonification;
générer (332), sur la base des premières données de réseau et des secondes données de réseau et de l'agencement spatial mutuel, une image composée correspondant à la région d'intérêt (221);
le procédé (300) comprenant en outre les étapes consistant à:
recevoir, à partir du premier réseau d'émetteursrécepteurs à ultrasons (210₁), des premières données de suivi de réseau correspondant à des signaux ultrasonores détectés par le premier réseau de transducteurs à ultrasons (210₁) en réponse à des signaux ultrasonores émis par le second réseau de transducteurs à ultrasons (210₂); ou
recevoir, à partir du second réseau émetteur-récepteur à ultrasons (210₂), des secondes données de suivi de réseau correspondant à des signaux ultrasonores détectés par le second réseau de transducteurs à ultrasons (210₂) en réponse à des signaux ultrasonores émis par le premier réseau de transducteurs à ultrasons (210₁); et
déterminer l'agencement spatial mutuel sur la base des premières données de suivi de réseau ou des secondes données de suivi de réseau de manière correspondante.

2. Produit de programme d'ordinateur selon la revendication 1, dans lequel la génération d'une image composée (332) comprend:
pondérer les premières données de réseau et des secondes données de réseau à des positions correspondantes dans la région d'intérêt (221); et
additionner les premières données de réseau pondérées et des secondes données de réseau pondérées aux positions correspondantes pour fournir l'image composée.

3. Produit de programme d'ordinateur selon la revendication 1, dans lequel la génération d'une image composée (332) comprend:
reconstruire une première image ultrasonore basée sur les premières données de réseau, la première image ultrasonore comprenant des premières données d'intensité d'image ultrasonore;
reconstruire une seconde image ultrasonore basée sur les secondes données de réseau, la seconde image ultrasonore comprenant des secondes données d'intensité d'image ultrasonore;
pondérer les premières données d'intensité d'image ultrasonore et les secondes données d'intensité d'image ultrasonore à des positions correspondantes dans la région d'intérêt (221); et
additionner les premières données d'intensité d'image ultrasonore pondérées et les secondes données d'intensité d'image ultrasonore pondérées aux positions correspondantes pour fournir l'image composée.

4. Produit de programme d'ordinateur selon la revendication 1, dans lequel la génération d'une image composée (332) comprend:
la comparaison des premières données de réseau et des secondes données de réseau à des positions correspondantes dans la région d'intérêt (221) et
la sélection de la plus grande valeur desdites données à chaque position correspondante pour fournir l'image composée.

5. Produit programme d'ordinateur selon l'une quelconque des revendications 1 à 4 dans lequel:
les premières données de réseau comprennent des signaux ultrasonores temporels correspondant à chacune d'une pluralité de lignes de balayage d'image au premier angle d'insonification (112_{1,n});
les secondes données de réseau comprennent des signaux ultrasonores temporels correspondant à chacune d'une pluralité de lignes de balayage d'image au second angle d'insonification (112_{2,n}) ;
dans lequel la génération d'une image composée (332) inclut le filtre passe-bande, à chacune d'une pluralité de fréquences centrales, des signaux ultrasonores temporels correspondant à chacune d'une pluralité de lignes de balayage d'image au premier angle d'insonification (112_{1,n}) et le calcul, pour chaque ligne de balayage d'image, d'une moyenne pondérée des signaux ultrasonores filtrés par filtre passe-bande;
le filtre passe-bande, à chacune d'une pluralité de fréquences centrales, des signaux ultrasonores temporels correspondant à chacune d'une pluralité de lignes de balayage d'image au second angle d'insonification (112_{2,n}) et le calcul, pour chaque ligne de balayage d'image, d'une moyenne pondérée des signaux ultrasonores filtrés par filtre passe-bande; et
additionner la moyenne pondérée des signaux ultrasonores filtrés par filtre passe-bande au premier angle d'insonification (112_{1,n}) et la moyenne pondérée des signaux ultrasonores filtrés par filtre passe-bande au second angle d'insonification (112_{2,n}) à des positions correspondantes dans la région d'intérêt (221) pour fournir l'image composée.

6. Produit de programme d'ordinateur selon la revendication 1, dans lequel les signaux ultrasonores émis par le second réseau de transducteurs à ultrasons (210₂) correspondent à au moins un lobe latéral de l'insonification de la région d'intérêt (221) par le second réseau de transducteurs à ultrasons (210₂) au second angle d'insonification (θ₂);
ou dans lequel les signaux ultrasonores émis par le premier réseau de transducteurs à ultrasons (210₁) correspondent à au moins un lobe latéral de l'insonification de la région d'intérêt (221) par le premier réseau de transducteurs à ultrasons au premier angle d'insonification (θ₁), de manière correspondante.

7. Produit de programme d'ordinateur selon les revendications 1 ou 6, dans lequel l'étape consistant à déterminer l'agencement spatial mutuel sur la base des premières données de suivi de réseau correspondantes ou des secondes données de suivi de réseau correspondantes, comprend:
pour les premières données de suivi de réseau, calculer au moins une distance entre le premier réseau émetteur-récepteur à ultrasons (210₁) et le second réseau émetteur-récepteur à ultrasons (210₂) sur la base d'un temps de vol des signaux ultrasonores détectés par le premier réseau de transducteurs à ultrasons (210₁) en réponse aux signaux ultrasonores émis par le second réseau de transducteurs à ultrasons (210₂), et
comprend pour les secondes données de suivi de réseau, le calcul d'au moins une distance entre le premier réseau émetteur-récepteur à ultrasons (210₁) et le second réseau émetteur-récepteur à ultrasons (210₂) sur la base d'un temps de vol des signaux ultrasonores détectés par le second réseau transducteur à ultrasons (210₂) en réponse aux signaux ultrasonores émis par le premier réseau transducteur à ultrasons (210₁).

8. Produit programme d'ordinateur selon la revendication 1 dans lequel:
les signaux ultrasonores émis par le second réseau de transducteurs à ultrasons (210₂) forment un front d'onde hémisphérique rayonnant vers l'extérieur par rapport au second réseau de transducteurs à ultrasons (210₂), dans lequel les premières données de suivi de réseau correspondent à des données de temps de vol indiquant une distance entre le second réseau de transducteurs à ultrasons (210₂) et chacun d'une pluralité d'éléments de réseau du premier réseau émetteur-récepteur à ultrasons (210₁), et
dans lequel l'agencement spatial mutuel est déterminé sur la base des premières données de suivi de réseau en triangulant la position de chacun de la pluralité d'éléments de réseau du premier réseau d'émetteursrécepteurs à ultrasons (210₁) par rapport au second réseau de transducteurs à ultrasons (210₂); ou
dans lequel les signaux ultrasonores émis par le premier réseau de transducteurs à ultrasons (Tx, 210₁) f forment un front d'onde hémisphérique rayonnant vers l'extérieur par rapport au premier réseau de transducteurs à ultrasons (Tx, 210₁), dans lequel les secondes données de suivi de réseau correspondent à des données de temps de vol indicatives d'une distance entre le premier réseau de transducteurs à ultrasons (Tx, 210₁) et chacun d'une pluralité d'éléments de réseau du second réseau d'émetteurs-récepteurs à ultrasons (RX_{1..3}, 210₂), et dans lequel l'agencement spatial mutuel est déterminé sur la base des secondes données de suivi de réseau en triangulant la position de chacun de la pluralité d'éléments de réseau du second réseau d'émetteurs-récepteurs à ultrasons (RX_{1..3}, 210₂) par rapport au premier réseau de transducteurs à ultrasons (Tx, 210₁), de manière correspondante.

9. Dispositif d'imagerie ultrasonore (600) comprenant:
un cathéter (220) d'échographie intra-cardiaque (ICE) comprenant un premier réseau d'émetteurs-récepteurs à ultrasons (210₁)
et un second réseau d'émetteurs-récepteurs à ultrasons (210₂) dans lequel le premier réseau d'émetteursrécepteurs à ultrasons (210₁) et le second réseau d'émetteurs-récepteurs à ultrasons (210₂) sont séparés axialement le long d'un axe de longueur (214) du cathéter ICE (220) de sorte que le premier réseau d'émetteurs-récepteurs à ultrasons (210₁) et le second réseau d'émetteurs-récepteurs à ultrasons (210₂) ont un agencement spatial mutuel; et
un processeur (622) configuré pour exécuter les étapes du procédé consistant à:
recevoir (330), à partir du premier réseau émetteur-récepteur à ultrasons (210₁), des premières données de réseau correspondant à des signaux ultrasonores détectés par le premier réseau transducteur à ultrasons (210₁) en réponse à une insonification d'une région d'intérêt (221) par le premier réseau transducteur à ultrasons (210₁) à un premier angle d'insonification (θ₁);
recevoir (331), à partir du second réseau émetteur-récepteur à ultrasons (210₂), des secondes données de réseau correspondant à des signaux ultrasonores détectés par le second réseau transducteur à ultrasons (210₂) en réponse à une insonification de la région d'intérêt (221) par le second réseau transducteur à ultrasons (210₂) à un second angle d'insonification (θ₂), différent du premier angle d'insonification;
générer (332), sur la base des premières données de réseau et des secondes données de réseau et de l'agencement spatial mutuel, une image composée correspondant à la région d'intérêt (221); le procédé (300) comprenant en outre les étapes consistant à:
recevoir, à partir du premier réseau d'émetteursrécepteurs à ultrasons (210₁), des premières données de suivi de réseau correspondant à des signaux ultrasonores détectés par le premier réseau de transducteurs à ultrasons (210₁) en réponse à des signaux ultrasonores émis par le second réseau de transducteurs à ultrasons (210₂);
ou recevoir, à partir du second réseau émetteur-récepteur à ultrasons (210₂), des secondes données de suivi de réseau correspondant à des signaux ultrasonores détectés par le second réseau de transducteurs à ultrasons (210₂) en réponse à des signaux ultrasonores émis par le premier réseau de transducteurs à ultrasons (210₁); et
déterminer l'agencement spatial mutuel sur la base des premières données de suivi de réseau ou des secondes données de suivi de réseau de manière correspondante.
